(19) Europäisches Patentamt

**European Patent Office**

**Office européen des brevets**

(11) Veröffentlichungsnummer: **0 170 871 B1**

# (12) EUROPÄISCHE PATENTSCHRIFT

(45) Veröffentlichungstag der Patentschrift: **08.04.92**

(51) Int. Cl.⁵: **A61K 7/16**

(21) Anmeldenummer: **85108106.7**

(22) Anmeldetag: **29.06.85**

Die Akte enthält technische Angaben, die nach dem Eingang der Anmeldung eingereicht wurden und die nicht in dieser Patentschrift enthalten sind.

(54) **Zahnpasta.**

(30) Priorität: **07.07.84 DE 3425152**

(43) Veröffentlichungstag der Anmeldung:
**12.02.86 Patentblatt 86/07**

(45) Bekanntmachung des Hinweises auf die Patenterteilung:
**08.04.92 Patentblatt 92/15**

(84) Benannte Vertragsstaaten:
**AT BE CH DE FR GB IT LI NL**

(56) Entgegenhaltungen:
**DE-A- 2 704 504**
**DE-A- 2 758 548**
**DE-A- 2 807 006**
**US-A- 3 957 968**

(73) Patentinhaber: **Henkel Kommanditgesellschaft auf Aktien**
**Postfach 1100 Henkelstrasse 67**
**W-4000 Düsseldorf-Holthausen(DE)**

(72) Erfinder: **Plöger, Walter, Dr.**
**Holbeinweg 11**
**W-4010 Hilden(DE)**
Erfinder: **Klüppel, Hans-Jürgen, Dr.**
**Begonienstr. 7**
**W-4000 Düsseldorf(DE)**
Erfinder: **Förg, Franz, Dr.**
**Rotdornweg 10**
**W-4018 Langenfeld(DE)**

EP 0 170 871 B1

Rank Xerox (UK) Business Services

**Beschreibung**

Gegenstand der Erfindung ist eine Zahnpasta mit hoher Reinigungs- und Polierwirkung bei gleichzeitig niedrigen Abrieb- und Kratzwerten.

Zahnpasten werden bei der täglichen Reinigung der Zähne durch Bürsten mit einer Zahnbürste angewendet. Die Zahnpasta soll in erster Linie die Reinigung der Zahnoberflächen von Speiseresten, Verfärbungen durch z.B. Tabak oder Tee und von den fest anhaftenden bakteriellen Zahnbelägen, der sogenannten Plaque, unterstützen. Dies geschieht hauptsächlich durch die in der Zahnpasta enthaltenen Poliermittel, in geringerem Maße auch durch die enthaltenen Tenside. Die Poliermittel müssen, um ihre Reinigungs- und Polierwirkung zu entfalten, eine gewisse Abrasivität gegenüber der Zahnoberfläche aufweisen. Es ist jedoch von größter Bedeutung, daß die Abrasivität gegenüber Zahnschmelz und Dentin auf niedrigen Werten gehalten wird, um eine Schädigung der Zahnoberfläche durch den täglichen Gebrauch der Zahnpasta zu vermeiden. Vor allem dürfen die verwendeten Poliermittel keine tiefen Kratzer auf der Zahnoberfläche verursachen. Erwünscht ist vielmehr eine Einebnung der ggf. vorhandenen Rauhigkeit der Zahnoberfläche.

Schließlich soll ein geeignetes Poliermittel mit den übrigen Komponenten der Zahnpasta verträglich sein. Es soll sich mit Wasser, Feuchthaltemitteln und Konsistenzreglern zu einer duktilen, aus Tuben oder Spendern leicht dosierbaren Paste verarbeiten lassen und die Wirkung von bekannten karieshemmenden Wirkstoffen, z.B. von Fluoridträgern wie NaF oder Na-Monofluorphosphat auch bei längerer Lagerung nicht mindern.

Es war, z.B. aus US-PS 3 957 968, bekannt, daß Zahnpasten die als Poliermittel eine Kombination aus Alpha-Aluminiumoxid und einem weicheren Poliermittel enthalten, eine gute Reinigungs- und Polierwirkung aufweisen. Es war aber auch bekannt, daß durch den Gehalt an Alpha-Aluminiumoxid (Korund) ein relativ hoher Zahnschmelzabrieb verursacht wird. Nach US-PS 4 144 322 wird vorgeschlagen, diesen Übelstand durch Zusatz von bestimmten Calcium-, Magnesium- oder Natriumsalzen und Einhalten eines pH-Wertes über 7 zu verringern. Nach US-PS 4 060 599 wird vorgeschlagen, das Alpha-Aluminiumoxid in einer ganz speziellen Teilchengrößenverteilung einzusetzen.

Aus DE-A-2807006 waren Zahnpflegemittel bekannt, die als Poliermittelkomponente synthetische, gefällte Kieselsäuren und gegebenenfalls kleinere Anteile an calciniertem Aluminiumoxid und gegebenenfalls zusätzlich Alpha-Aluminium-Trihydrat enthalten. Der Calcinationsgrad des calcinierten Aluminiumoxids ist nicht angegeben.

Die vorgenannten Maßnahmen haben das Problem der zu hohen Schmelzabrasion und vor allem das der zu starken Kratzwirkung dieser Poliermittel nicht befriedigend gelöst.

Ein Ziel der vorliegenden Erfindung war daher das Auffinden geeigneter Poliermittel oder Poliermittelkombinationen, die zwar eine optimale Reinigung und Entfärbung der Zahnoberfläche bewirken, aber nur geringe Abrasivität und Kratzwirkung aufweisen. Ein weiteres Ziel der Erfindung war das Auffinden eines für diese Poliermittel geeigneten Trägers, der die Herstellung einer Zahnpasta von geeigneter Konsistenz für die Dosierung aus Tuben und Spendern und von hoher Stabilität gegen Änderung der Konsistenz bei Lagerung ermöglicht. Schließlich war es ein Ziel der vorliegenden Erfindung, eine Zahnpasta zu finden, die aufgrund ihrer gesamten Zusammensetzung die vielfachen Anforderungen an ein modernes Zahnpflegemittel in optimaler Weise erfüllt.

Diese Aufgaben wurden erfindungsgemäß gelöst durch eine Zahnpasta, umfassend 15 - 30 Gew.-% einer Poliermittelkombination aus Kieselsäure-Poliermitteln (A) und Aluminiumoxid-Poliermitteln (B), 70 - 85 Gew.-% eines Trägers aus Wasser, Feuchthaltemitteln und Konsistenzreglern und bis zu 5 Gew.-% weiterer üblicher Zahnpastenzusätze, dadurch gekennzeichnet, daß als Kieselsäure-Poliermittel (A) eine Gelkieselsäure und/oder eine Fällungskieselsäure und als Aluminiumoxid-Poliermittel (B) eine schwach calcinierte Tonerde, bestehend aus 10 - 50 Gew.-% Gamma-Aluminiumoxid ($\gamma$-$Al_2O_3$) und 50 - 90 Gew.-% Alpha-Aluminiumoxid ($\alpha$-$Al_2O_3$) enthalten ist und die Poliermittelkomponenten im Gewichtsverhältnis A : B = 100 : (2 - 15) enthalten sind.

Die schwach calcinierte Tonerde hat bevorzugt einen Gehalt von ca. 20 Gew.-% Gamma-Aluminiumoxid ($\gamma$-$Al_2O_3$) und ca. 80 Gew.-% Alpha-Aluminiumoxid ($\alpha$-$Al_2O_3$), eine Agglomeratgröße unter 20 $\mu$m, eine mittlere Primärkristallgröße von 0,5 - 1,5 $\mu$m und ein Schüttgewicht von 500 - 600 g/l.

Geeignete schwach calcinierte Tonerden werden durch Calcination aus Aluminiumhydroxyd hergestellt. Aluminiumhydroxid geht durch Calcination in das bei Temperaturen oberhalb 1 200 °C thermodynamisch stabile $\alpha$-$Al_2O_3$ über. Die bei Temperaturen zwischen 400 und 1 000 °C auftretenden, thermodynamisch instabilen $Al_2O_3$-Formen bezeichnet man als Gamma-Formen (vgl. Ullmann, Encyclopädie der technischen Chemie, 4. Auflage (1974) Band 7, Seite 298). Durch Wahl der Temperatur und der Zeitdauer bei der Calcination kann man den Calcinationsgrad, d.h. die Umwandlung in das thermodynamisch stabile $\alpha$-$Al_2O_3$

2

auf beliebige Höhe einstellen. Man erhält durch schwache Calcination eine Tonerde mit einem Gehalt an $\gamma$-$Al_2O_3$ der um so niedriger ist, je höher die Calcinationstemperatur und je länger die Calcinationsdauer gewählt wird. Schwach calcinierte Tonerden unterscheiden sich von reinem $\alpha$-$Al_2O_3$ durch eine geringere Härte der Agglomerate, eine größere spezifische Oberfläche und größere Porenvolumina.

Der Dentinabrieb (RDA) der erfindungsgemäß zu verwendenden schwächer calcinierten Tonerden mit einem Anteil von 10 - 50 Gew.-% $\gamma$-$Al_2O_3$ beträgt nur 30 - 60 % des Dentinabriebs eines stark calcinierten, reinen $\alpha$-$Al_2O_3$ (gemessen in einer Standard-Zahnpaste mit 20 Gew.-% Tonerde als einzigem Poliermittel).

Im Gegensatz zum $\alpha$-$Al_2O_3$ läßt sich das $\gamma$-$Al_2O_3$ mit einer wässrig-ammoniakalischen Lösung von Alizarin S (1,2-Dihydroxy-9,10-anthrachinon-4-sulfonsäure) rot anfärben. Man kann den Grad der Anfärbbarkeit als Maß für den Calcinationsgrad bzw. für den Anteil an $\gamma$-$Al_2O_3$ in einer calcinierten Tonerde wählen:

ca. 1 g $Al_2O_3$, 10 ml einer Lösung von 2 g/l Alzarin S in Wasser und 3 Tropfen einer wässrigen, 10 Gew.-%igen Lösung von $NH_3$ werden in ein Reagenzglas gegeben und kurz aufgekocht. Das $Al_2O_3$ wird anschließend abfiltriert, nachgewaschen, getrocknet und unter dem Mikroskop beurteilt oder farbmetrisch ausgewertet.

Geeignete, schwach calcinierte Tonerden mit einem Gehalt von 10 - 50 Gew.-% $\gamma$-$Al_2O_3$ lassen sich nach diesem Verfahren schwach bis tief rosa anfärben.

Aluminiumoxid-Poliermittel verschiedener Calcinationsgrade, Mahlfeinheit und Schüttgewichte sind im Handel erhältlich, z.ß. die "Poliertonerden" der Firma Giulini-Chemie.

Geeignete Kieselsäure-Poliermittel sind z.B. die Gelkieselsäuren, die durch Umsetzung von Natriumsilikatlösungen mit starken wässrigen Mineralsäuren unter Ausbildung eines Hydrosols, Alterung zum Hydrogel, Waschen und Trocknen erzeugt werden. Erfolgt die Trocknung unter schonenden Bedingungen auf Wassergehalte von 15 - 35 Gew.-%, so werden die sogenannten Hydrogelkieselsäuren erhalten, wie sie z.B. aus US-PS 4 153 680 bekannt sind.

Durch Trocknung auf Wassergehalte von unterhalb 15 Gew.-% erfolgt eine irreversible Schrumpfung der vorher lockeren Struktur des Hydrogels zur dichten Struktur des sogen. Xerogels. Solche Xerogelkieselsäuren sind z.B. in US-PS 3 538 230 beschrieben.

Die Hydrogel-Kieselsäuren sind bevorzugte Kieselsäure-Poliermittel für die erfindungsgemäßen Zahnpasten. Dies gilt besonders für solche mit einem Wassergehalt von 15 - 35 Gew.-% und einer Partikelgröße von 0,5 - 30 $\mu$, wobei wenigstens 80 Gew.-% der Primärpartikel unter 5 $\mu$ liegen sollten.

Xerogel-Kieselsäuren sind wegen ihrer größeren Härte und Abrasivität in geringeren Anteilen als Komponente in der Poliermittel-Mischung zur Einstellung der gewünschten Polierwirkung geeignet.

Eine zweite, bevorzugt geeignete Gruppe von Kieselsäure-Poliermitteln sind die Fällungskieselsäuren. Diese werden durch Ausfällung von Kieselsäure aus verdünnten Alkalisilikat-Lösungen durch Zugabe von starken Säuren unter Bedingungen erhalten, bei welchen die Aggregation zum Sol und Gel nicht eintreten kann. Geeignete Verfahren zur Herstellung von Fällungskieselsäuren sind z.B. in DE-OS 25 22 486 und in DE-OS 31 14 493 beschrieben. Bevorzugt geeignet ist eine gemäß DE-OS 31 14 493 hergestellte Fällungskieselsäure mit einer BET-Oberfläche von 15 - 110 $m^2/g$, einer Partikelgröße von 0,5 - 30 $\mu m$, wobei wenigstens 80 Gew.-% der Primärpartikel unter 5 $\mu m$ liegen sollen, und einer Viskosität in 30%iger Glycerin-Wasser-(1 : 1)-Dispersion von 30 - 60 Pa.s (20 °C).

Die erfindungsgemäßen Zahnpflegemittel weisen aufgrund der speziellen Poliermittelkombination ein hervorragendes Reinigungsvermögen auch gegenüber Zahnverfärbungen durch Tee und Nikotin auf. Gleichzeitig wird bei nur mäßigem Dentin- und Schmelzabrieb eine hohe Polierwirkung (Einebnung von Aufrauhungen) erzielt. Trotz des Gehaltes an einer relativ harten Poliermittelkomponente - der schwach calcinierten Tonerde, weisen die erfindungsgemäßen Zahnpasten praktisch keine Kratzwirkung auf.

Diese Ergebnisse sind als durchaus überraschend anzusehen, da nach bisheriger Erfahrung eine hohe Reinigungs- und/oder Polierwirkung durch höheren Dentin- oder Schmelzabrieb und/oder höhere Kratzwerte erkauft werden mußte.

Für Spezialzahnpasta - z.B. Kinderzahncremes, Raucherzahncremes, Zahncremes gegen Zahnverfärbungen, die nur gelegentlich angewendet werden usw. - ,kann das gewünschte Ausmaß an Reinigungs- und Polierwirkung und an noch tolerablen Abriebswerten durchaus unterschiedlich sein. Stets wird jedoch durch die erfindungsgemäßen Zahnpasten ein, im Hinblick auf die erzielte Reinigungs- und Polierwirkung, sehr niedriger Abrieb erzielt. Die Einstellung snezieller Polier- und Abriebswerte ist einerseits durch den Calcinierungsgrad der Tonerde, d.h. den Anteil des Gamma-Aluminiumoxids in der Tonerde und den Anteil an Tonerde am Poliermittel andererseits auch durch den Einsatz von verschiedenen Kieselsäurequalitäten und Mischungen solcher Kieselsäure-Poliermittel möglich.

Zur Herstellung einer für den täglichen Gebrauch geeigneten sogenannten Raucherzahncreme, die somit die Zahnverfärbungen, herrührend aus dem Genuß von Tabak bzw. aber auch von Tee schonend bekämpft, hat sich der Einsatz einer Poliermittelkombination aus

| 12 - 20 Gew.-% | einer Hydrogel-Kieselsäure |
| 1 - 5 Gew.-% | einer Xerogel-Kieselsäure und |
| 0,2 - 5 Gew.-% | einer schwach calcinierten Tonerde mit 10 - 50 Gew.-% $\gamma$-Al$_2$O$_3$ und 50 - 90 Gew.-% $\alpha$-Al$_2$O$_3$ |

jeweils bezogen auf die gesamte Zahnpaste, als besonders reinigungseffektiv erwiesen.

Als Träger für die erfindungsgemäßen Zahnpasten, der die Herstellung von Pasten mit geeigneter Konsistenz für die Abfüllung in bzw. die Dosierung aus Tuben und Spendern auf Grundlage der erfindungsgemäßen Poliermittelkombination ermöglicht, eignet sich eine Kombination aus Wasser, Feuchthaltemitteln und Konsistenzreglern. Als Feuchthaltemittel können z.B. Glycerin, Sorbit, Xylit, Propylenglycole, Polyethylenglycole, insbesondere solche mit mittleren Molekulargewichten von 200 - 800 eingesetzt werden. Als Konsistenzregler (bzw. Bindemittel) dienen z.B. natürliche und/oder synthetische wasserlösliche Polymere wie Karragheenate, Traganth, Stärke und Stärkeether, Celluloseether wie z.B. Carboxymethylcellulose (Na-Salz), Hydroxyethylcellulose, Methylhydroxypropylcellulose, Guar, Akaziengum, Agar-Agar, Xanthangum, Johannisbrotmehl, Pectine, wasserlösliche Carboxyvinylpolymere (z.B. Carbopol$^R$-Typen), Polyvinylalkohol, Polyvinylpyrrolidon, Polyethylenglycole, insbesondere solche mit Molekulargewichten von 1 500 - 1 000 000.

Weitere Stoffe, die sich zur Viskositätskontrolle eignen, sind z.B. Schichtsilikate wie z.B. Montmorillonit-Tone, kolloidale Verdickungskieselsäuren wie z.B. Aerogel-Kieselsäuren oder pyrogene Kieselsäuren. Ein für die Herstellung von Zahnpasten mit der erfindungsgemäßen Poliermittelkombination besonders gut geeigneter Träger enthält

| 25 - 35 Gew.-% | Wasser |
| 25 - 35 Gew.-% | Sorbit |
| 10 - 15 Gew.-% | Glycerin |
| 2 - 10 Gew.-% | Polyethylenglycol (mittleres Molekulargewicht 200 - 800) |
| 0,1 - 0,5 Gew.-% | Carboxymethylcellulose und |
| 1 - 3 Gew.-% | Verdickungskieselsäure (z.B. Syloblanc$^R$ 34) |

jeweils bezogen auf die gesamte Zahnpasta.

Weitere übliche Zahnpastenzusätze, die insgesamt bis zu ca. 5 Gew.-% der Zahnpastenzusammensetzung ausmachen können, sind z.B. Tenside zur Unterstützung der Reinigungswirkung und ggf. zur Entwicklung von Schaum beim Zähnebürsten und zur Stabilisierung der Dispersion der Poliermittelkomponenten in dem Träger. Geeignete Tenside sind z.B. lineare Natriumalkylsulfate mit 12 - 18 C-Atomen in der Alkylgruppe. Diese Stoffe weisen zusätzlich eine enzymhemmende Wirkung auf den bakteriellen Stoffwechsel des Zahnbelags auf. Weitere geeignete Tenside sind Alkalisalze, bevorzugt Natriumsalze von Alkylpolyglycolethersulfat mit 12 - 16 C-Atomen in der linearen Alkylgruppe und 2 - 6 Glycolethergruppen im Molekül, von Alkyl (C$_{12}$ – $_{16}$)-benzolsulfonat, von linearem Alkan(C$_{12}$ – $_{18}$)-sulfonat, von Sulfobernsteinsäuremonoalkyl(C$_{12}$ – C$_{18}$)-estern, von sulfatierten Fettsäuremonoglyceriden, sulfatierten Fettsäurealkanolamiden, Sulfoessigsäurealkyl(C$_{12}$ – C$_{16}$)-estern, Acylsarcosinen, Acyltauriden und Acylisethionaten mit jeweils 8 - 18 C-Atomen in der Acylgruppe. Auch nichtionische Tenside sind geeignet, z.B. Oxethylate von Fettsäuremono- und -diglyceriden, von Fettsäure-Sorbitanestern und Ethylenoxid/Propylenoxid-Blockpolymerisate.

Weitere übliche Zahnpastenzusätze sind
- Konservierungsmittel und antimikrobielle Stoffe, wie z.B. p-Hydroxybenzoesäuremethyl-, -ethyl- oder propylester, Natriumsorbat, Natriumbenzoat, Bromchlorophen, Phenyl-salicylsäureester, Thymol usw.
- Antizahnsteinwirkstoffe, z.B. Organophosphonate wie die Natriumsalze von 1-Hydroxyethan-1,1-diphosphonsäure, 1-Phosphonopropan-1,2,3-tricarbonsäure und andere Phosphonsäuren, wie sie z.B. aus US-PS 3 488 419, DE-OS 22 24 430 und DE-OS 23 43 196 bekannt sind,
- Karieshemmende Stoffe wie z.B. Natriumfluorid, Natriummonofluorphosphat, Zinnfluorid
- Süßungsmittel wie z.B. Saccharin-Natrium, Natrium-Cyclamat, Sucrose, Lactose, Maltose, Fructose,
- Aromen wie z.B. Pfefferminzöl, Krauseminzöl, Eukalyptusöl, Anisöl, Fenchelöl, Kümmelöl, Menthylacetat, Zimtaldehyd, Anethol, Vanillin, Thymol sowie Mischungen dieser und anderer natürlicher und synthetischer Aromen,
- Pigmente wie z.B. Titandioxid
- Farbstoffe
- Puffersubstanzen wie z.B. primäre, sekundäre oder tertiäre Alkaliphosphate oder Citronensäure/Natriumcitrat,
- Wundheilende und entzündungshemmende Stoffe wie z.B. Allantoin, Harnstoff sowie Azulen, Kamillewirkstoffe, Acetylsalicylsäurederivate.

Die erfindunsgemäßen Zahnpasten enthalten bevorzugt

4

0,5 - 3,0 Gew.-%     anionischer Tenside, z.B. Natriumlaurylsulfat

0,1 - 1,0 Gew.-%     Natriumfluorid und/oder Natriummonofluorphosphat und

0,1 - 0,3 Gew.-%     Saccharin-Natrium.

| Zahncreme | Beispiele | |
|---|---|---|
| | 1 | 2 |
| Fällungskieselsäure[1] | 18 Gew.-% | -- |
| Hydrogelkieselsäure[2] | -- | 15 Gew.-% |
| Xerogelkieselsäure[3] | -- | 4 |
| schwach calcinierte Tonerde[4] | 1,0 | 1,0 |
| verdickungskieselsäure (pyrogen)[5] | 0,8 | 0;5 |
| Sorbit | 17,5 | 17,5 |
| Glycerin | 17,5 | 17,5 |
| Carboxymethylcellulose[6] | 0,9 | 0,6 |
| Natrium-laurylsulfat[7] | 2,0 | 2,0 |
| Natriumfluorid | 0,22 | 0,22 |
| Saccharin-Natrium | 0,2 | 0,2 |
| Aromaöle | 1,0 | 1,0 |
| Wasser, Konservierungsmittel | ad 100 | ad 100 |

[1] Sident[R] 12 DS, Firma DEGUSSA

[2] Syloblanc[R] 84, Firma GRACE

[3] Syloblanc[R] 81, Firma GRACE

[4] Poliertonerde P10 feinst, Firma Giulini-Chemie

[5] Aerosil[R] 200, Firma DEGUSSA

[6] Relatin[R] 100 S 8, Firma HENKEL KGaA

[7] Texapon[R] K 1296, Firma HENKEL KGaA

**Patentansprüche**

1. Zahnpaste, umfassend 15 - 30 Gew.-% einer Poliermittelkombination aus Kieselsäure-Poliermitteln und Aluminiumoxid-Poliermitteln, 70 - 85 Gew.-% eines Trägers aus Wasser, Feuchthaltemitteln und Konsistenzreglern und bis zu 5 Gew.-% weiterer übliche Zahnpastenzusätze, dadurch gekennzeichnet, daß als Kieselsäure-Poliermittel (A) eine Gelkieselsäure und/oder eine Fällungskieselsäure und als Aluminiumoxid-Poliermittel (B) eine schwach calcinierte Tonerde, bestehend aus 10 - 50 Gew.-% Gamma-Aluminiumoxid ($\gamma$-$Al_2O_3$) und 50 - 90 Gew.-% Alpha-Aluminiumoxid ($\alpha$-$Al_2O_3$) enthalten ist und die Poliermittelkomponenten im Gewichtsverhältnis A : B = 100 : (2 - 15) enthalten sind.

2. Zahnpaste nach Anspruch 1, dadurch gekennzeichnet, daß als Aluminiumoxid-Poliermittel (B) eine schwach calcinierte Tonerde mit einem Gehalt an ca. 20 Gew.-% Gamma-Aluminiumoxid ($\gamma$-$Al_2O_3$) und ca. 80 Gew.-% Alpha-Aluminiumoxid mit einer Agglomeratgröße unter 20 $\mu$m und einer mittleren Primärkristallgröße von 0,5 - 1,5 $\mu$m enthalten ist.

3. Zahnpaste nach Anspruch 1 und 2, dadurch gekennzeichnet, daß als Kieselsäure-Poliermittel (A) eine Hydrogelkieselsäure mit einen Wassergehalt von 15 - 35 Gew.-% und einer Partikelgröße von 0,5 - 30 $\mu$m, wobei wenigstens 80 Gew.-% der Primärpartikel unter 5 $\mu$m liegen, enthalten ist.

4. Zahnpaste nach Anspruch 1 oder 2, dadurch gekennzeichnet, daß als Kieselsäure-Poliermittel (A) eine Fällungskieselsäure mit einer BET-Oberfläche von 15 - 110 m²/g, einer Partikelgröße von 0,5 - 30 $\mu$m, wobei wenigstens 80 Gew.-% der Primärpartikel unter 5 $\mu$m liegen, enthalten ist.

5. Zahnpaste nach Anspruch 1 oder 2, dadurch gekennzeichnet, daß als Poliermittelkombination

    12 - 20 Gew.-%     einer Hydrogelkieselsäure

    1 - 5 Gew.-%     einer Xerogelkieselsäure und

    0,5 - 2 Gew.-%     der schwach calcinierten Tonerde

jeweils bezogen auf die gesamte Zahnpaste, enthalten sind.

**6.** Zahnpaste nach Anspruch 1 - 5, dadurch gekennzeichnet, daß als Träger

| | |
|---|---|
| 25 - 35 Gew.-% | Wasser |
| 25 - 35 Gew.-% | Sorbit |
| 10 - 15 Gew.-% | Glycerin |
| 1 - 10 Gew.-% | Polyethylenglycol (mittleres Molgewicht 200 - 800) |
| 0,1 - 0,5 Gew.-% | Carboxymethylcellulose und |
| 1 - 3 Gew.-% | Verdickungskieselsäure |

jeweils bezogen auf die gesamte Zahnpaste, enthalten sind.

**Claims**

1. A toothpaste containing from 15 to 30% by weight of a polishing agent combination of silica polishes and aluminium oxide polishes, from 70 to 85% by weight of a carrier of water, humectants and consistency regulators and up to 5% by weight of other standard toothpaste ingredients, characterized in that a silica gel and/or a precipitated silica is/are present as the silica polish (A) and a weakly calcined alumina, consisting of 10 to 50% by weight of gamma-aluminium oxide ($\gamma$-$Al_2O_3$) and 50 to 90% by weight of alpha-aluminium oxide ($\alpha$-$Al_2O_3$), as the aluminium oxide polish (B), the ratio by weight between the polishing components A and B amounting to 100:(2-15).

2. A toothpaste as claimed in claim 1, characterized in that a weakly calcined alumina containing approximately 20% by weight of gamma-aluminium oxide ($\gamma$-$Al_2O_3$) and approximately 80% by weight of alpha-aluminium oxide with an agglomerate size of less than 20 $\mu$m and an average primary crystal size of from 0.5 to 1.5 $\mu$m is present as the aluminium oxide polish (B).

3. A toothpaste as claimed in claims 1 and 2, characterized in that a silica hydrogel containing from 15 to 35% by weight of water and having a particle size of 0.5 to 30 $\mu$m, at least 80% by weight of the primary particles being smaller than 5 $\mu$m is present as the silica polish (A).

4. A toothpaste as claimed in claim 1 or 2, characterized in that a precipitated silica having a BET surface of 15 to 110 m$^2$/g and a particle size of 0.5 to 30 $\mu$m, at least 80% by weight of the primary particles being smaller than 5 $\mu$m, is present as the silica polish (A).

5. A toothpaste as claimed in claim 1 or 2, characterized in that

| | |
|---|---|
| 12 to 20% by weight | silica hydrogel |
| 1 to 5% by weight | silica xerogel and |
| 0.5 to 2% by weight | weakly calcined alumina, |

based in each case on the toothpaste as a whole, are present as the polishing agent combination.

6. A toothpaste as claimed in claims 1 to 5, characterized in that

| | |
|---|---|
| 25 to 35 % by weight | water, |
| 25 to 35 % by weight | sorbitol, |
| 10 to 15 % by weight | glycerol |
| 1 to 10 % by weight | polyethylene glycol (average molecular weight 200-800) |
| 0.1 to 0.5% by weight | carboxymethyl cellulose and |
| 1 to 3 % by weight | thickening silica, |

based in each case on the toothpaste as a whole, are present as the carrier.

**Revendications**

1. Pâte dentifrice, comprenant 15 à 30 % en poids d'une association d'agents polissants, se composant d'agents polissants à base d'acide silicique et d'agents polissants à base d'oxyde d'aluminium, 70 à 85 % en poids d'un vecteur constitué d'eau, de préservateurs d'humidité et de régulateurs de consistance, et jusqu'à 5 % en poids d'autres additifs usuels de pâtes dentifrices, caractérisée en ce que sont contenus comme agent polissant à base d'acide silicique (A), un acide silicique en gel et/ou un acide silicique précipité, et comme agent polissant à base d'oxyde d'aluminium (B), une alumine faiblement calcinée se composant de 10 à 50 % en poids d'oxyde d'aluminium gamma ($\gamma$-$Al_2O_3$) et de 50 à 90 % en poids d'oxyde d'aluminium alpha ($\alpha$-$Al_2O_3$), et en ce que les composants des agents polissants sont contenus dans un rapport pondéral de A : B = 100 : (2 - 15).

2. Pâte dentifrice selon la revendication 1, caractérisée en ce qu'est contenu comme agent polissant à base d'oxyde d'aluminium (B), une alumine légèrement calcinée contenant environ 20 % en poids d'oxyde d'aluminium gamma ($\gamma$-Al$_2$O$_3$) et environ 80 % en poids d'oxyde d'aluminium alpha avec une grosseur d'aggloméré inférieure à 20 $\mu$m et une grosseur de cristal primaire moyenne de 0,5 à 1,5 $\mu$m.

3. Pâte dentifrice selon les revendications 1 et 2, caractérisée en ce qu'est contenu comme agent polissant à base d'acide silicique (A), un acide silicique en hydrogel dont la teneur en eau est comprise entre 15 et 35 % et dont la grosseur de particule va de 0,5 à 30 $\mu$m 80 % en poids au moins des particules primaires étant inférieurs à 5 $\mu$m.

4. Pâte dentifrice selon la revendication 1 ou 2, caractérisée en ce qu'est contenu comme agent polissant à base d'acide silicique (A), un acide silicique précipité avec une surface BET comprise entre 15 et 110 m$^2$/g et une grosseur de particule allant de 0,5 à 30 $\mu$m, 80 % en poids au moins des particules primaires étant inférieurs à 5 $\mu$m.

5. Pâte dentifrice selon la revendication 1 ou 2, caractérisée en ce que sont contenus comme association d'agents polissants
   12 à 20 % en poids d'un acide silicique en hydrogel
   1 à 5 % en poids d'un acide silicique en xérogel
   0,5 à 2 % en poids de l'alumine légèrement calcinée, dans chaque cas par rapport à la totalité de la pâte dentifrice.

6. Pâte dentifrice selon les revendications 1 à 5, caractérisée en ce que sont contenus comme vecteurs
   25 à 35 % en poids d'eau
   25 à 35 % en poids de sorbitol
   10 à 15 % en poids de glycérine
   1 à 10 % en poids de polyéthylèneglycol (poids moléculaire moyen: 200 à 800)
   0,1 à 0,5 % en poids de carboxyméthylcellulose et
   1 à 3 % en poids d'acide silicique épaississant,
   dans chaque cas par rapport à la totalité de la pâte dentifrice.